# EUROPEAN PATENT APPLICATION

(11) **EP 4 484 430 A1**
(43) Date of publication of application: **01.01.2025**
(21) Application number: 23759285.2
(22) Date of filing: 24.02.2023
(51) Int. Cl.: C07D 487/04, A61K 31/519, A61P 19/02

(54) **POLYMORPH OF JAK TYROSINE KINASE INHIBITOR AND PREPARATION METHOD THEREFOR**

(30) Priority: 24.02.2022 CN 202210173823
(71) Applicant: Prime Gene Therapeutics Co., Ltd., Beijing 100176 (CN)
(72) Inventor: MA, Sufeng, Beijing 100176 (CN); LUO, Huasong, Beijing 100176 (CN); HAN, Junru, Beijing 100176 (CN); HU, Wei, Beijing 100176 (CN); ZHU, Li, Beijing 100176 (CN)
(74) Representative: Høiberg P/S
(86) International application number: PCT/CN2023/078243
(87) International publication number: WO 2023/160663

(57) **Abstract**

The present disclosure relates to a polymorph of a compound 2-{3-[3-amino-4-(7H-pyrrolo[2, 3-d]pyrimidine-4-yl)-1H-pyrazole-1-yl]-1-(isopropylsulfonyl)azetidine-3-yl}acetonitrile (formula A), and a pharmaceutical composition and a use thereof.

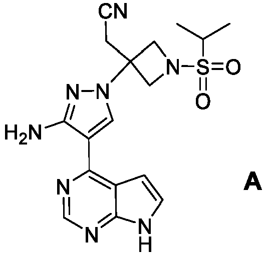

## Description

### Technical Field

This disclosure belongs to the field of chemical drugs and relates to a polymorph of chemical drugs, in particular relates to a polymorph of the compound 2-{3-[3-amino-4-(7*H*-pyrrolo[2,3-*d*]pyrimidin-4-yl)-1*H*-pyrazol-1-yl]-1-(isopropylsulfonyl)azeti din-3-yl}acetonitrile and preparation methods thereof.

### Background Art

2-{3-[3-amino-4-(7*H*-pyrrolo[2,3-*d*]pyrimidin-4-yl)-1*H*-pyrazol-1-yl]-1-(isopropylsulfonyl) azetidin-3-yl}acetonitrile (compound A) belongs to small molecular inhibitors of the JAK family of non-receptor tyrosine kinase, and its JAK-STAT signaling pathway is closely related to inflammatory cytokines and tumors, and is widely involved in important biological processes such as cell proliferation, differentiation, transfer apoptosis, regulation of immune response and cell homeostasis. Compound A has the structure represented by the following formula: 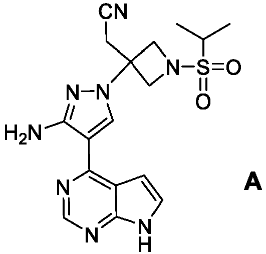 The synthesis method of this compound is disclosed in the patent CN201711248509.8 which, however, does not involve the crystal form of the compound. None of other literature report on crystal forms of 2-{3-[3-amino-4-(7*H*-pyrrolo[2,3- d] pyrimidin-4-yl)-1H-pyrazol-1-yl]-1-(isopropylsulfonyl) azetidin-3-yl} acetonitrile. It is well known that polymorphs of a drug are of great significance to the physical properties, bioavailability, and quality of the drug as well as the process for preparing the drug. The differences in physical and chemical properties between different crystal forms of a drug affect the stability of the drug. The bioavailability of the same drug may be significantly different due to different crystal forms, and different crystal forms affect the dissolution rate of the drug. Moreover, the difference in surface free energy of different crystal forms will cause different binding forces between crystal particles, which will affect the fluidity, particle uniformity, content uniformity and physical stability of the drug. Therefore, it is necessary to study the crystal forms of a drug.

### Summary

The purpose of this disclosure is to provide a polymorph of compound A and its preparation method. The chemical name of compound A is 2-{3-[3-amino-4-(7*H*-pyrrolo[2,3-d] pyrimidin-4-yl)-1H-pyrazol-1-yl]-1-(isopropylsulfonyl)azetidin-3-yl}acetonitrile. The synthesis method of the compound A is disclosed in the patent CN201711248509.8, and in this disclosure, compound A was prepared with reference to the patent for preparing the polymorphs thereof.

According to one aspect, the disclosure provides a polymorph of Compound A.

The disclosure provides a polymorph of compound A, which includes various crystal forms, such as crystal form I, crystal form IIa, crystal form IIb, and crystal form III; and also provides crystal forms of salts of compound A.

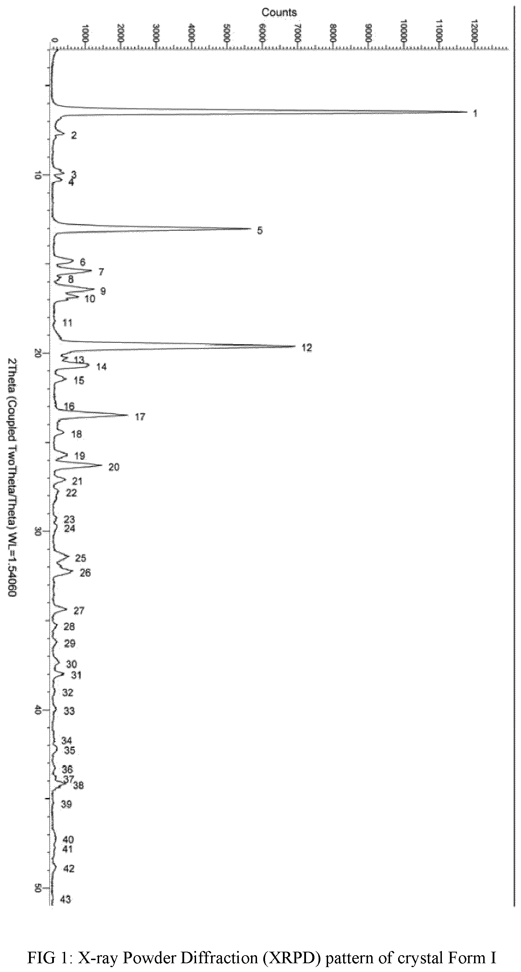

The X-ray powder diffraction pattern of 2-{3-[3-amino-4-(7*H*-pyrrolo[2,3-d] pyrimidin-4-yl)-1H-pyrazol-1-yl]-1-(isopropylsulfonyl)azetidin-3-yl}acetonitrile crystal form I is shown in FIG 1, wherein the measurement error of 2θ is ±0.2 degrees, and there are multiple characteristic peaks between 0~40 degrees, as shown in the following table.

**Table 1: X-ray diffraction data of crystal form I**

| **2θ** | **d- (Å)** | **Relative intensity (100%)** |
|---|---|---|
| 6.48 | 13.62 | 100.0% |
| 7.68 | 11.49 | 2.7 |
| 9.90 | 8.92 | 2.8 |
| 13.01 | 6.79 | 47.7 |
| 14.81 | 5.97 | 4.5 |
| 15.38 | 5.75 | 8.9 |
| 16.43 | 5.38 | 9.4 |
| 16.87 | 5.25 | 5.5 |
| 19.61 | 4.52 | 58.0 |
| 20.68 | 4.29 | 8.2 |
| 23.47 | 3.78 | 17.6 |
| 26.29 | 3.38 | 11.4 |
| 32.23 | 2.77 | 4.7 |

The crystal form I of this disclosure shows no exothermic and endothermic peaks between 50°C and 200°C in differential scanning calorimetry (DSC), and there are endothermic peaks near 213.5°C, 219.6°C and 229.0°C, and obvious exothermic peaks between 213.5°C~229.0°C. The differential scanning calorimetry curve of crystal form I indicates that the crystal form will undergo crystal transformation during the process of heating and melting. It is manifested that after the crystal form is endothermic and melted at about 213.5 °C, it is exothermic and transformed into a more stable crystal form. When the temperature rises further, the newly formed crystal form undergoes endothermic melting. The transformation of the crystal form shows the relative magnitude of the stability (melting point, lattice energy) between different crystal forms, but this change occurs at a higher temperature (>200.0°C) and does not correspond to the stability of the crystal form under conventional use conditions.

The crystal form I of the present disclosure does neither contain crystallization water nor contain crystallization solvent.

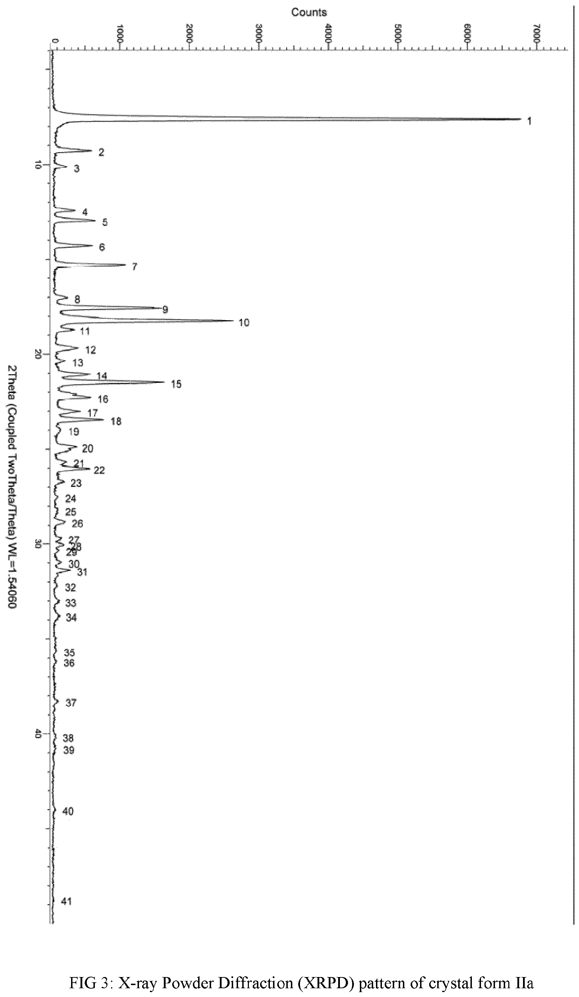

This disclosure also provides 2-{3-[3-amino-4-(7*H*-pyrrolo[2,3-d] pyrimidin-4-yl)-1H-pyrazol-1-yl]-1-(isopropylsulfonyl)azetidin-3-yl}acetonitrile crystal form IIa. The X-ray powder diffraction pattern of crystal form IIa is shown in FIG 3, where the measurement error of 2θ is ± 0.2 degrees, and the multiple characteristic peaks contained in 0~40 degrees are shown in the following table:

**Table 2: X-ray diffraction data of crystal form IIa**

| **2θ** | **d- (Å)** | **Relative intensity (100%)** |
|---|---|---|
| 7.61 | 11.59 | 100.0% |
| 9.28 | 9.51 | 7.8 |
| 12.44 | 7.10 | 4.5 |
| 12.96 | 6.82 | 8.8 |
| 14.28 | 6.19 | 8.1 |
| 15.30 | 5.78 | 15.0 |
| 17.56 | 5.04 | 21.4 |
| 18.23 | 4.86 | 37.6 |
| 21.05 | 4.51 | 6.7 |
| 21.47 | 4.13 | 22.7 |
| 22.28 | 3.98 | 6.9 |
| 23.45 | 3.79 | 9.9 |
| 24.87 | 3.57 | 4.0 |
| 26.03 | 3.41 | 6.6 |

The crystal form IIa of this disclosure shows no endothermic peaks between 50-200°C and shows endothermic peaks around 220.2°C in differential scanning calorimetry (DSC).

The crystal form IIa does not contain crystallization water and is an anhydrous substance.

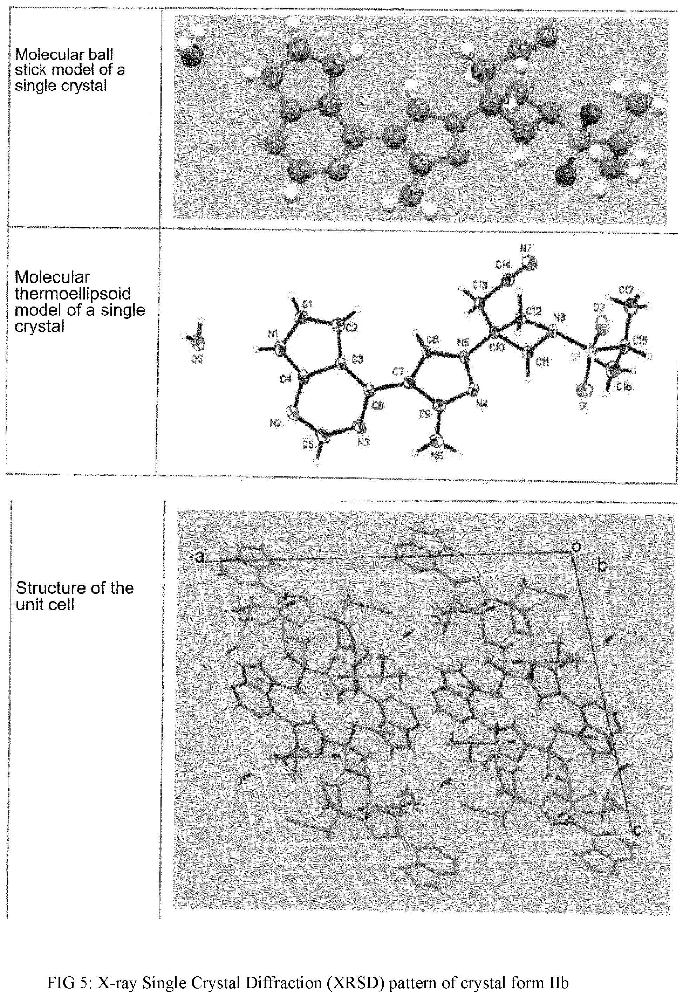

This disclosure additionally provides 2-{3-[3-amino-4-(7*H*-pyrrolo[2,3-d] pyrimidin-4-yl)-1H-pyrazol-1-yl]-1-(isopropylsulfonyl)azetidin-3-yl}acetonitrile crystal form IIb. The X-ray powder diffraction pattern of crystal form IIb is shown in FIG 5, wherein the measurement error of 2θ is ± 0.2 degree, and there are multiple characteristic peaks between 0~40 degree as shown in the following table.

**Table 3: X-ray diffraction data of crystal form IIb**

| **2θ** | **d- (Å)** | **Relative intensity (100%)** |
|---|---|---|
| 7.93 | 11.13 | 100.0% |
| 9.36 | 9.43 | 4.1 |
| 11.29 | 7.82 | 2.9 |
| 14.69 | 6.02 | 18.9 |
| 14.95 | 5.91 | 13.1 |
| 15.13 | 5.85 | 4.8 |
| 15.95 | 5.55 | 13.3 |
| 19.93 | 4.44 | 9.7 |
| 20.15 | 4.40 | 6.9 |
| 20.96 | 4.23 | 4.9 |
| 21.36 | 4.15 | 12.3 |
| 21.77 | 4.07 | 10.3 |
| 22.12 | 4.01 | 5.1 |
| 22.79 | 3.89 | 11.7 |
| 23.54 | 3.77 | 5.4 |
| 24.14 | 3.68 | 7.9 |
| 26.38 | 3.37 | 2.6 |
| 28.74 | 3.10 | 2.1 |
| 29.15 | 3.06 | 3.7 |
| 31.69 | 2.82 | 2.8 |

The crystal form IIb of this disclosure shows endothermic peaks between 50-200°C and endothermic peaks around 221.5°C in differential scanning calorimetry (DSC).

The crystalline form IIb shows a weight loss of about 2.2% at 50-150°C in thermogravimetric analysis (TGA). According to the weight loss calculation, the weight loss is about 0.5 molecules of water, therefore, the crystal form IIb is a hydrate of compound A with 0.5 molecules of water.

According to another aspect, the present disclosure relates to an oral pharmaceutical composition comprising the crystal form I of the present disclosure.

According to yet another aspect, the present disclosure relates to a pharmaceutical composition for topical use, which comprises the crystal form IIb of the present disclosure.

According to yet another aspect, the present disclosure relates to the use of the above-mentioned crystal forms or the pharmaceutical compositions of the compound A in the preparation of a medicament for treating diseases mediated by Janus kinase.

According to another aspect, the present disclosure relates to crystal form I or crystal form IIb, for use in the treatment of diseases mediated by Janus kinase.

According to yet another aspect, the present disclosure relates to a method for treating diseases mediated by Janus kinase, which comprises administering the above-mentioned crystal forms or pharmaceutical compositions of the compound A to an individual in need thereof.

### Description of Drawings

FIG 1: X-ray Powder Diffraction (XRPD) pattern of crystal Form I.

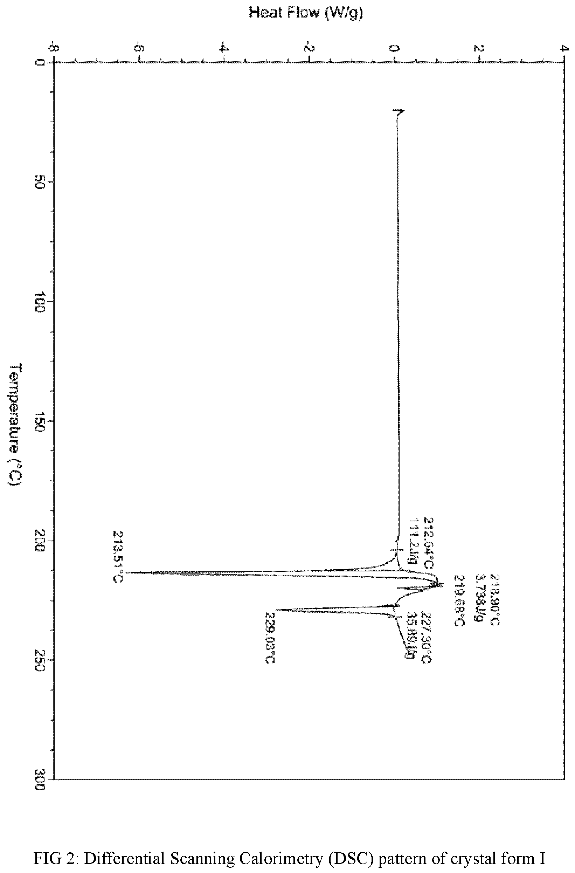

FIG 2: Differential Scanning Calorimetry (DSC) pattern of crystal form I.
FIG 3: X-ray Powder Diffraction (XRPD) pattern of crystal form IIa.

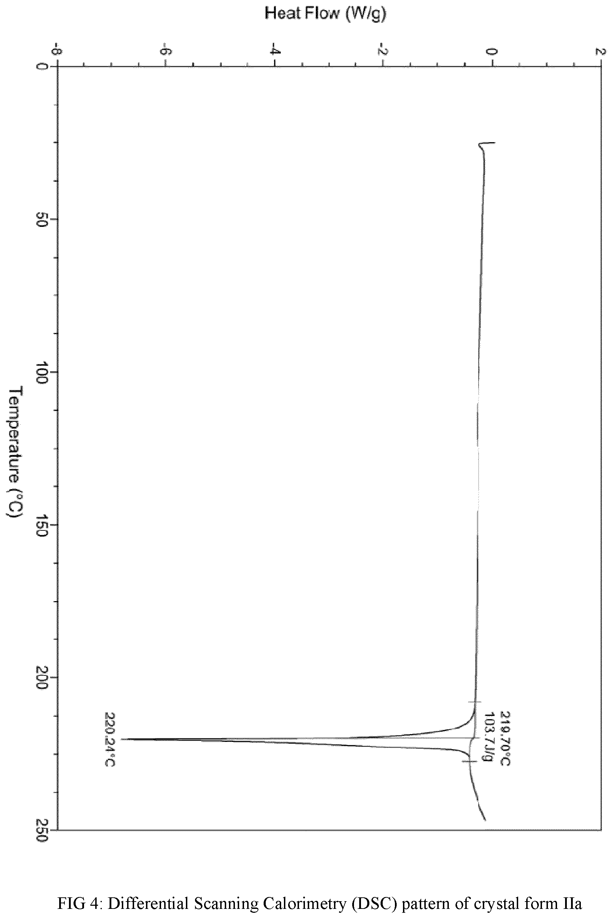

FIG 4: Differential Scanning Calorimetry (DSC) pattern of crystal form IIa.
FIG 5: X-ray Single Crystal Diffraction (XRSD) pattern of crystal form IIb.

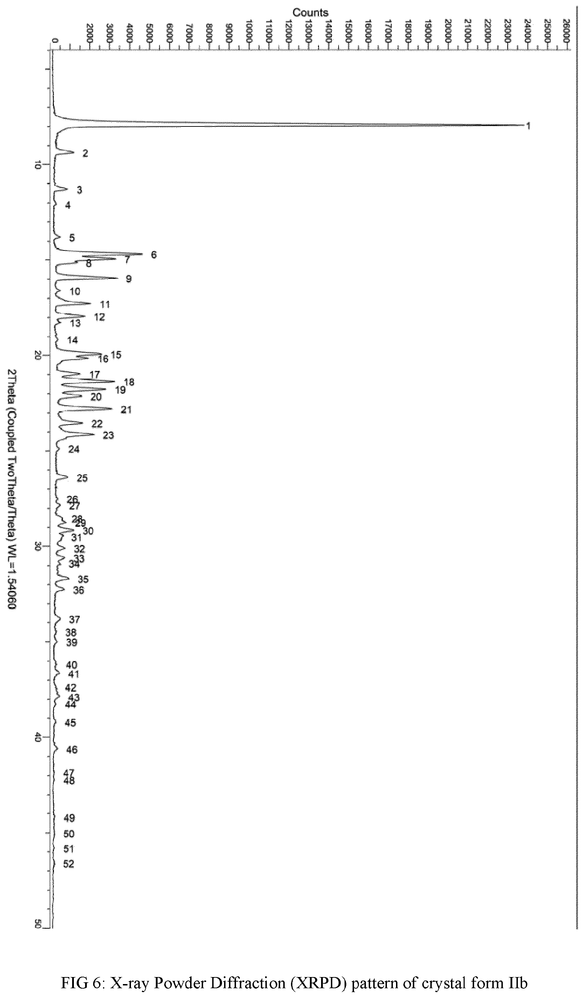

FIG 6: X-ray Powder Diffraction (XRPD) pattern of crystal form IIb.

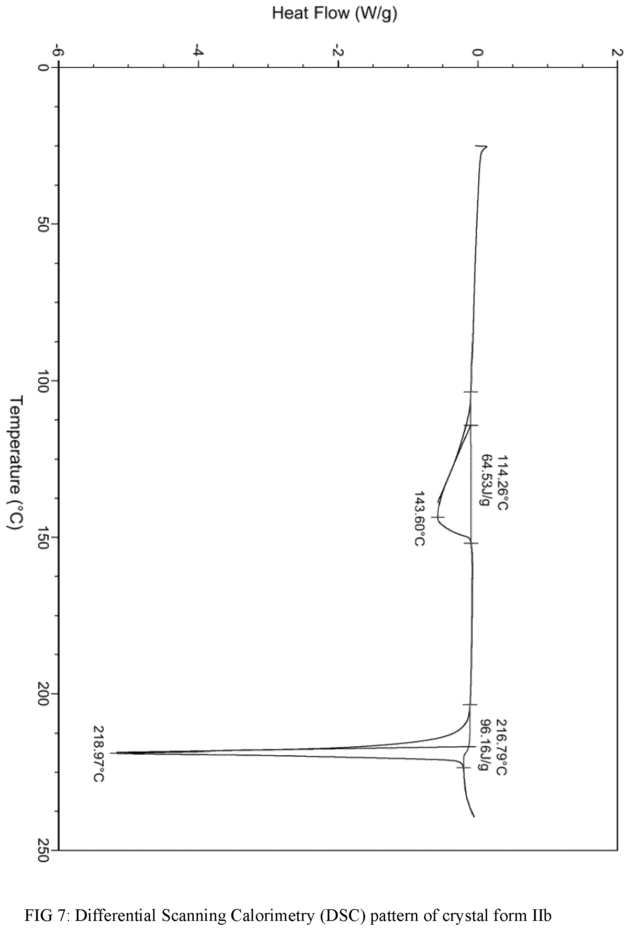

FIG 7: Differential Scanning Calorimetry (DSC) pattern of crystal form IIb.

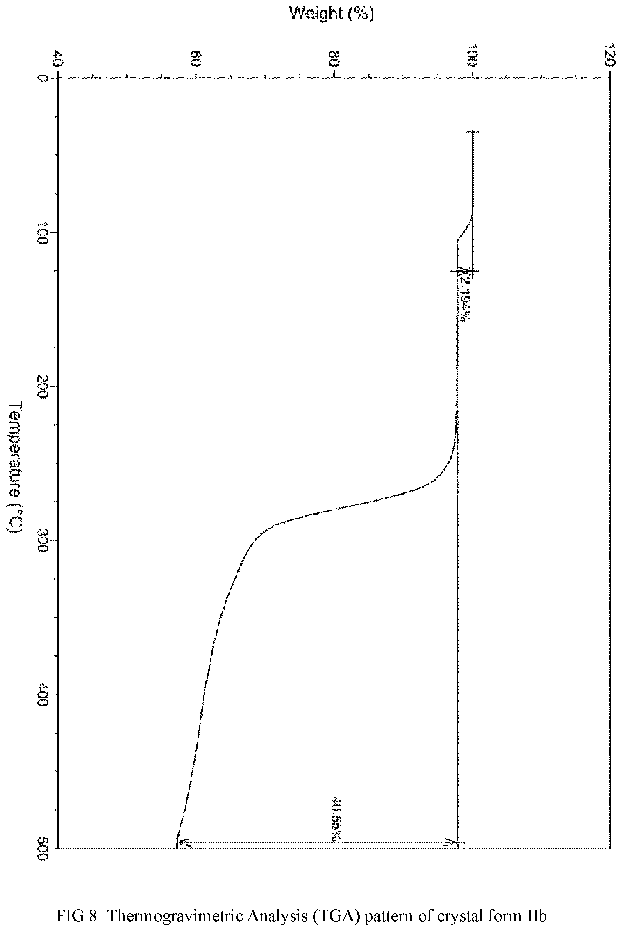

FIG 8: Thermogravimetric Analysis (TGA) pattern of crystal form IIb.
FIG 9: Transdermal rate of crystal form I and crystal form IIb in transdermal test.

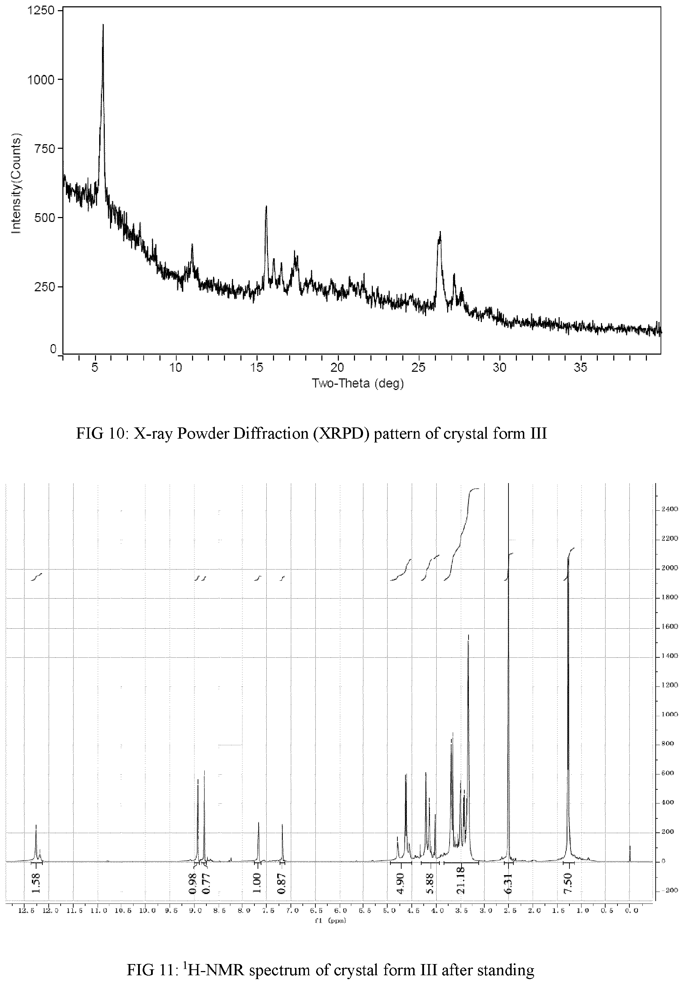

FIG 10: X-ray Powder Diffraction (XRPD) pattern of crystal form III.
FIG 11: ¹H-NMR spectrum of crystal form III after standing.

### Embodiments

In the following, various embodiments of the present disclosure will be described in more detail with reference to examples, so that a person of ordinary skill in the art can better understand the present disclosure and its advantages. It should be noted that the following embodiments give a further non-restrictive detailed description of the technical solutions of the present disclosure, which should not be regarded as a limitation on the scope of the present disclosure, but only an exemplary description and typical representative of the present disclosure.

The reagents used in these examples, unless otherwise specified, are chemically pure reagents, and the supplier is Fuchen (Tianjin) Chemical Reagent Co., Ltd. The experimental instrument used in the examples, including a round-bottom flask, a Brinell funnel, a suction filter flask, a magnetic stirring instrument (DF-101S) are all laboratory general instruments, and the drying equipment is a vacuum oven (DZF-6020 type) or a double cone (FZG-8 type).

### Example 1

### Preparation of 2-{3-[3-amino-4-(7H-pyrrolo[2,3-d] pyrimidin-4-yl)-1H-pyrazol-1-yl]-1-(isopropylsulfonyl) azetidin-3-yl} acetonitrile crystal form I

In a 250mL flask, add 20.0g of the compound 2- f 3-[3-amino-4-(7H-pyrrolo[2,3-d]pyrimidin-4-yl)-1H-pyrazol-1-yl]-1-(isopropylsulfonyl)azetidin-3-yl}acetonitrile and 100mL N,N-dimethylformamide, stir to dissolve, filter, filter out insoluble matter, add 250mL of purified water to the filtrate, stir to crystallize, filter, and after the obtained solid was dried and transfer the dried solid into a 250mL flask, continue to add 200mL of absolute ethanol, heat up and reflux for 3h, cool, filter, and thus obtained solid was dried to constant weight at 55-60 °C to render 18.8g of off-white solid, with a yield of 94.0% and a chemical purity of 99.62%.

### Example2

### Preparation of 2-{3-[3-amino-4-(7H-pyrrolo[2,3- d] pyrimidin-4-yl)-1H-pyrazol-1-yl]-1-(isopropylsulfonyl) azetidin-3-yl} acetonitrile phosphate

2-{3-[3-amino-4-(7*H*-pyrrolo[2,3-d]pyrimidin-4-yl)-1H-pyrazol-1-yl]-1-(isopropylsulfonyl) azetidin-3-yl} acetonitrile (400mg, 1.0mmol, 1.0eq) was added in 20mL of acetonitrile, then 85% phosphoric acid (350mg, 3.0mmol, 3.0eq) was added at room temperature, reacted overnight at room temperature, concentrated under reduced pressure to obtain oily product which is not salted.

### Example 3

### Preparation of 2-{3-[3-amino-4-(7H-pyrrolo[2,3- d] pyrimidin-4-yl)-1H-pyrazol-1-yl]-1-(isopropylsulfonyl) azetidin-3-yl} acetonitrile crystal form IIa

In a 100mL flask, add 5.0g of 2-{3-[3-amino-4-(7*H*-pyrrolo[2,3-d]pyrimidin-4-yl)-1*H*-pyrazol-1-yl]-1-(Isopropylsulfonyl)azetidin-3-yl}acetonitrile crystal form I, add ethanol (95%) 100mL, stir with heating and reflux for 5h, cool down and filter to obtain a solid, which was dried at 80 °C for 12h under vacuum (0.1MPa), and 4.8g of solid was obtained, with a yield of 96.0% and a purity of 99.50% measured by HPLC.

### Example 4

### Preparation of 2-{3-[3-amino-4-(7H-pyrrolo[2,3- d] pyrimidin-4-yl)-1H-pyrazol-1-yl]-1-(isopropylsulfonyl) azetidin-3-yl} acetonitrile phosphate

2-{3-[3-amino-4-(7*H*-pyrrolo[2,3- d] pyrimidin-4-yl)-1H-pyrazol-1-yl]-1-(isopropylsulfonyl) azetidin-3-yl} acetonitrile (400mg, 1.0mmol, 1.0eq) was added in 20mL of acetonitrile, 85% phosphoric acid (350mg, 3.0mmol, 3.0eq) was added at room temperature, and then the temperature was raised to 70°C and reacted for 3 hours. HPLC showed that the reaction system became complicated (the area of the main peak changed from 98% to 87%). After concentrated under reduced pressure, it was obtained an oily substance without salt formation.

### Example 5

### Preparation of 2-{3-[3-amino-4-(7H-pyrrolo[2,3- d] pyrimidin-4-yl)-1H-pyrazol-1-yl]-1-(isopropylsulfonyl) azetidin-3-yl} acetonitrile hydrochloride

2-{3-[3-amino-4-(7*H*-pyrrolo[2,3- d] pyrimidin-4-yl)-1*H*-pyrazol-1-yl]-1-(isopropylsulfonyl) azetidin-3-yl} acetonitrile (400mg, 1.0mmol, 1.0eq) was added to 20mL of acetonitrile, and then 38% concentrated hydrochloric acid (290mg, 3.0mmol, 3.0eq) was added at room temperature, and reacted overnight at room temperature. HPLC showed impurities increased. After concentrated under reduced pressure, an oily substance was obtained without salt formation.

### Example 6

### Preparation of 2-{3-[3-amino-4-(7H-pyrrolo[2,3- d] pyrimidin-4-yl)-1H-pyrazol-1-yl]-1-(isopropylsulfonyl) azetidin-3-yl} acetonitrile crystal form IIb

In a 100mL flask, add 6.2g of 2-{3-[3-amino-4-(7*H*-pyrrolo[2,3-d] pyrimidin-4-yl)-1*H*-pyrazol-1-yl]-1-(isopropylsulfonyl) azetidin-3-yl} acetonitrile and 25mL N,N-dimethylformamide, stir to dissolve, filter, filter out insoluble matter, add 50mL of purified water to the filtrate, stir and crystallize, filter, and dry the obtained solid to constant weight at 55-60 °C, 5.6g of solid was obtained with a yield of 88.9%.

### Example 7

### Preparation of 2-{3-[3-amino-4-(7H-pyrrolo[2,3- d] pyrimidin-4-yl)-1H-pyrazol-1-yl]-1-(isopropylsulfonyl) azetidin-3-yl} acetonitrile hydrochloride

2-{3-[3-amino-4-(7*H*-pyrrolo[2,3- d] pyrimidin-4-yl)-1*H*-pyrazol-1-yl]-1-(isopropylsulfonyl) azetidin-3-yl} acetonitrile (400mg, 1.0 mmol, 1.0eq) was added to 20mL of acetonitrile, 38% concentrated hydrochloric acid (290mg, 3.0mmol, 3.0eq) was added at room temperature, and the temperature was raised to 40°C and reacted for 3 hours. HPLC showed that the impurities in the reaction system increased. After concentrating under reduced pressure, an oily substance without salt formation was obtained.

### Example 8

### Preparation of 2-{3-[3-amino-4-(7H-pyrrolo[2,3- d] pyrimidin-4-yl)-1H-pyrazol-1-yl]-1-(isopropylsulfonyl) azetidin-3-yl} acetonitrile crystal form III

15 mg of 2-{3-[3-amino-4-(7*H*-pyrrolo[2,3- d] pyrimidin-4-yl)-1*H*-pyrazol-1-yl]-1-(isopropylsulfonyl) azetidin-3-yl} acetonitrile was added to 3mL of dioxane, then heated to 40°C to dissolve and the obtained solution is clear. After filtration, the solution was volatilized and crystallized at 40°C to obtain crystal form III. The crystal form III has poor crystallinity and stability, and the nuclear magnetic resonance showed that about 10% of impurities were generated after standing.

### Solubility investigation of different crystal forms

| **solubility in 20% propylene glycol at 32°C (µg/mL)** | | |
|---|---|---|
| **solvent** | **Example 1** | **Example 6** |
| 10minutes | 12.7 | 6.1 |
| 30minutes | 26.9 | 9.5 |
| 1 hour | 35.8 | 10.8 |
| 2 hours | 42.1 | 11.2 |
| 18 hours | 20.2 | 13.3 |

| **solubility measurement (37°C, 24 h)** | | |
|---|---|---|
| **pH** | **Example 1** | **Example 6** |
| pH | concentration (mg/mL) | concentration (mg/mL) |
| 1.0 | 0.4642 | 0.1499 |
| 2.0 | 0.0378 | 0.0174 |
| 3.8 | 0.0023 | 0.0009 |
| 4.5 | 0.0018 | 0.0007 |
| 5.5 | 0.0016 | 0.0005 |
| 6.8 | 0.0012 | 0.0006 |
| 7.0 | 0.0013 | 0.0008 |
| 8.0 | 0.0012 | 0.0007 |

### Stability investigation of different crystal forms

The crystals prepared in examples 1, 3 and 6 were selected, and the stability of the crystal forms I, IIa and IIb was investigated in accordance with the stability research guidelines issued by the National Medical Product Administration. The crystals were packed in pharmaceutical low-density polyethylene bags, and 24 months long-term stability was investigated. The results were as follows:
Long-term (24-months) stability of Compound A Crystal Form I

| Sample | Time | Appearance | Crystal Form | Loss on Drying | Related Substances (%) | | Content |
|---|---|---|---|---|---|---|---|
| | (Month) | | | (%) | Most Unknown Impurity | Total Impurities | (%) |
| Example 1 | 0 | Off-white powder | I | 0.08 | 0.17 | 0.58 | 99.9 |
| | 3 | Off-white powder | I | 0.06 | 0.16 | 0.57 | 100.1 |
| | 6 | Off-white powder | I | 0.08 | 0.17 | 0.57 | 100.0 |
| | 9 | Off-white powder | I | 0.11 | 0.17 | 0.57 | 99.8 |
| | 12 | Off-white powder | I | 0.09 | 0.17 | 0.55 | 99.9 |
| | 18 | Off-white powder | I | 0.12 | 0.18 | 0.62 | 99.7 |
| | 24 | Off-white powder | I | 0.10 | 0.16 | 0.59 | 99.8 |

Long-term (24-months) stability of Compound A Crystal Form IIa

| Sample | Time | Appearance | Crystal Form | Loss on Drying | Related Substances (%) | | Content |
|---|---|---|---|---|---|---|---|
| | (Month) | | | (%) | Most Unknown Impurity | Total Impurities | (%) |
| Example 3 | 0 | Off-white powder | IIa | 0.06 | 0.06 | 0.28 | 99.8 |
| | 3 | Off-white powder | IIb | 0.18 | 0.08 | 0.28 | 97.6 |
| | 6 | Off-white powder | IIb | 0.19 | 0.11 | 0.27 | 97.8 |
| | 9 | Off-white powder | IIb | 0.24 | 0.06 | 0.24 | 97.9 |
| | 12 | Off-white powder | IIb | 0.20 | 0.09 | 0.22 | 97.8 |
| | 18 | Off-white powder | IIb | 0.16 | 0.07 | 0.26 | 98.0 |
| | 24 | Off-white powder | IIb | 0.25 | 0.08 | 0.29 | 97.8 |

Long-term (24-months) stability of Compound A Crystal Form IIb

| Sample | Time | Appearance | Crystal Form | Loss on Drying | Related Substances (%) | | Content |
|---|---|---|---|---|---|---|---|
| | (Month) | | | (%) | Most Unknown Impurity | Total Impurities | (%) |
| Example 6 | 0 | Off-white powder | IIb | 0.36 | 0.07 | 0.38 | 99.7 |
| | 3 | Off-white powder | IIb | 0.36 | 0.08 | 0.38 | 99.7 |
| | 6 | Off-white powder | IIb | 0.39 | 0.08 | 0.37 | 99.6 |
| | 9 | Off-white powder | IIb | 0.34 | 0.08 | 0.38 | 99.5 |
| | 12 | Off-white powder | IIb | 0.30 | 0.07 | 0.37 | 99.6 |
| | 18 | Off-white powder | IIb | 0.31 | 0.07 | 0.39 | 99.6 |
| | 24 | Off-white powder | IIb | 0.32 | 0.08 | 0.39 | 99.7 |

The stability investigation results showed that 2-{3-[3-amino-4-(7*H*-pyrrolo[2,3- d] pyrimidin-4-yl)-1*H*-pyrazol-1-yl]-1-(isopropylsulfonyl) azetidin-3-yl} acetonitrile crystal forms I and IIb have better chemical stability. The crystal form IIa gradually absorbed water during standing and was transformed into crystal form lib. As a result, the crystal decreased in content due to weight increased by absorbing water.

### Transdermal Test

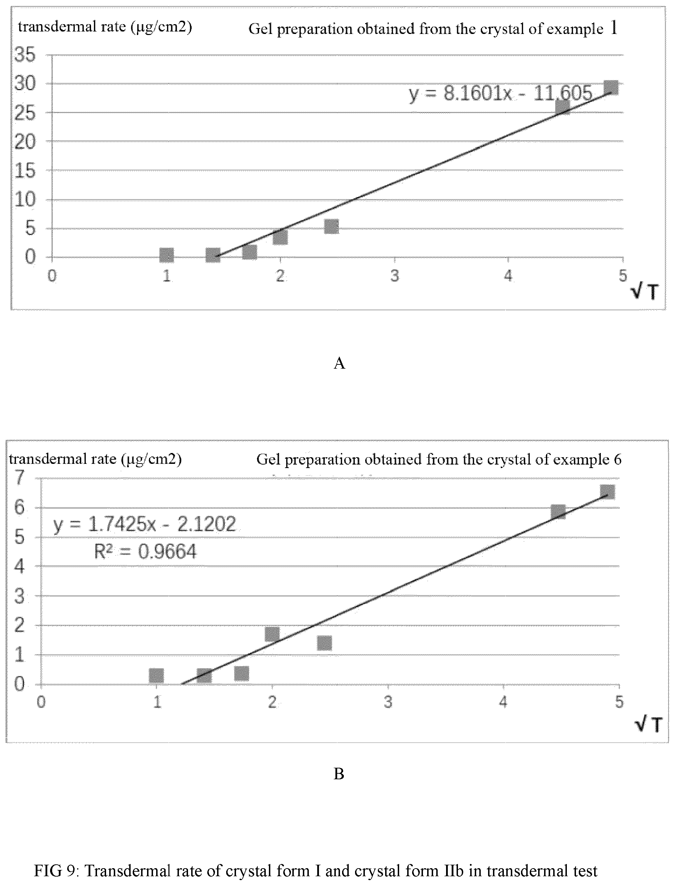

Take the crystal obtained in Examples 1 or 6 and prepare it into gel preparation (the process for preparing the gel preparation can be: after stirring PEG400, propylene glycol, glycerin, ethanol and the crystal of Example 1 or 6, mix with EDTA aqueous solution, add in the swelled carbomer aqueous solution, adjust pH to be 6 with 10% triethanolamine solution, to obtain the gel). Measure respective transdermal rate (receiving medium 5.0% Tween 80, pH5.5 phosphate). The test results are shown in FIG 9A and FIG 9B, which show that the crystal form I has better transdermal performance.

### Pharmacokinetic Experiment

The solid powders of Examples 1 and 6, and the hydrogel preparations thereof were taken, and the PK values of rats administered by oral gavage and transdermal application were respectively determined. Male SD rats were divided into groups, 3 rats in each group, and were administered the compounds obtained in Examples 1 and 6 by oral gavage (oral group), administered Example 1 intravenously (intravenous group), and administered the gel preparations prepared from the compounds of Examples 1 and 6 topically to the skin (transdermal group). Animals were fasted overnight before the experiment, and the fasting time ranged from 10 hours before administration to 4 hours after administration. Blood was collected at 0.25, 0.5, 1, 2, 4, 6, 8 and 24 hours after administration in oral group and transdermal group, and at 0.083, 0.25, 0.5, 1, 2, 4, 6, 8 and 24 hours after administration in intravenous group. After being anesthetized with isoflurane using a small animal anesthesia machine, 0.3 mL of whole blood was collected through the fundus venous plexus and placed in a heparin anticoagulant tube. The blood samples were centrifuged at 4°C and 4000 rpm for 5 min, and plasma was transferred to a centrifuge tube and placed at -80°C until analysis. Plasma was extracted by protein precipitation method, and the extract was analyzed by LC/MS/MS. The transdermal group was administered for 24hours and the skin at the administration site was peeled off after washing off the epidermal drug, and the drug content in the skin was analyzed by LC/MS/MS after homogenization. The results of pharmacokinetic experiments are shown in Table 1 and Table 2.

**Table 1. The pharmacokinetic parameters of male rats after administration of the compounds of the examples**

| | Intravenous group | Example 1 Oral group | Example 6 Oral group | Example 1 gel Transdermal group | Example 6 gel Transdermal group |
|---|---|---|---|---|---|
| T_{1/2} (hr) | 2.49 | 5.33 | 3.40 | 4.33 | ND |
| Tmax (hr) | 842 | 2.67 | 3.17 | 4.83 | 6.00 |
| Cmax (ng/mL) | / | 111 | 114 | 33.6 | 38.2 |
| Vss (L/kg) | 4.93 | | | | |
| Cl (mL/min/kg) | 69.2 | | | | |
| AUC_{0-inf}(hr*ng/mL) | 496 | 941 | 537 | 225 | 249 |
| F(%) | | 37 | 22 | 10 | 11 |

**Table 2. Drug concentration in the skin after topical administration to skin for 24 hours**

| | Example 1 gel Transdermal group | Example 6 gel Transdermal group |
|---|---|---|
| Mean value of drug concentration in rat skin (µg/g) | -80 | ~600 |

Crystal form I and crystal form IIb have the characteristics of high crystallinity and good stability. Meanwhile, crystal form I has higher solubility and oral bioavailability than crystal form lib. Although crystal form IIb is inferior to crystal form I in transdermal absorption, but the drug concentration retained in animal skin is significantly higher than that of the crystal form I by several times (for example, up to 7 times). This unexpected feature makes it more suitable for the application with less permeability but higher drug retention concentration in the skin.

## Claims

1. 2-{3-[3-amino-4-(7*H*-pyrrolo[2,3-*d*]pyrimidin-4-yl)-1*H*-pyrazol-1-yl]-1-(isopropylsulfo nyl)azetidin-3-yl}acetonitrile crystal form I, wherein the 2θ values of the characteristic peaks in the X-ray powder diffraction pattern of the crystal form I are 6.48, 13.01, 15.38, 19.61, 20.68, 23.47, and 26.29, with the measurement error of ± 0.2 degrees.

2. The crystal form I according to Claim 1, wherein the 2θ values of the characteristic peaks in the X-ray powder diffraction pattern of the crystal form are 6.48, 7.68, 9.90, 13.01, 14.81, 15.38, 16.43, 16.87, 19.61, 20.68, 23.47, 26.29 and 32.23, with the measurement error of ±0.2 degrees.

3. The crystal form I according to Claim 1, wherein the X-ray powder diffraction pattern of the crystal form is as shown in FIG 1.

4. 2-{3-[3-amino-4-(7*H*-pyrrolo[2,3- d] pyrimidin-4-yl)-1*H*-pyrazol-1-yl]-1-(isopropylsulfonyl) azetidin-3-yl} acetonitrile crystal form IIb, wherein the 2θ values of the characteristic peaks in the X-ray powder diffraction pattern of the crystal form IIb are 7.93, 9.36, 11.29, 14.95, 20.96, 21.36, 21.77 , 22.12, and 22.79, with the measurement error of ±0.2 degrees.

5. The crystal form IIb according to Claim 4, wherein the 2θ values of the characteristic peaks in the X-ray powder diffraction pattern of the crystal form IIb are 7.93, 9.36, 11.29, 14.69, 14.95, 15.13, 15.95, 19.93, 20.15, 20.96, 21.36, 21.77, 22.12, 22.79, 23.54, 24.14, 26.38, 28.74, and 29.15, with the measurement error of ±0.2 degrees.

6. The crystal form IIb according to Claim 4, wherein the X-ray powder diffraction pattern of the crystal form is as shown in FIG 6.

7. The crystal form IIb according to any of Claims 4 to 6, wherein the crystal form IIb is a crystal form of the hemihydrate of 2-{3-[3-amino-4-(7*H*-pyrrolo[2,3- d] pyrimidin-4-yl)-1*H*-pyrazol-1-yl]-1-(isopropylsulfonyl) azetidin-3-yl} acetonitrile.

8. An oral pharmaceutical composition comprising the crystal form I according to any of Claims 1 to 3.

9. A topical pharmaceutical composition comprising the crystal form IIb according to any of Claims 4 to 7.

10. Use of the crystal forms or pharmaceutical compositions according to any of Claims 1-9 in the preparation of a medicament for treating diseases mediated by Janus kinase.
